# EUROPEAN PATENT APPLICATION

(11) **EP 2 404 897 A1**
(43) Date of publication of application: **11.01.2012**
(21) Application number: 10748888.4
(22) Date of filing: 04.03.2010
(51) Int. Cl.: C07C 237/42, C07C 29/147, C07C 33/46, C07C 231/08, C07B 61/00

(54) **METHOD FOR PRODUCING HALOGEN-SUBSTITUTED BENZENE DIMETHANOL**

(30) Priority: 05.03.2009 JP 2009051677
(71) Applicant: Sumitomo Chemical Company, Limited, Tokyo 104-8260 (JP)
(72) Inventor: HAGIYA, Koji, Ibaraki-shi Osaka 567-0833 (JP); AOYAGI, Yasutaka, Oita-shi Oita 870-0832 (JP)
(74) Representative: Duckworth, Timothy John
(86) International application number: PCT/JP2010/054070
(87) International publication number: WO 2010/101299

(57) **Abstract**

A method for producing a halogen-substituted benzene dimethanol, the method including:
a first step of reacting a compound represented by formula (1) with a compound represented by formula (2) to obtain a dicarboxamide compound represented by formula (3), and
a second step of reducing the dicarboxamide compound using a borohydride compound to obtain a halogen-substituted benzene dimethanol represented by formula (4).

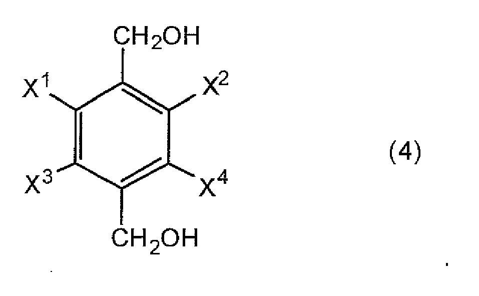

## Description

### TECHNICAL FIELD

The present invention relates to a method for producing a halogen-substituted benzene dimethanol.

### BACKGROUND ART

As a method for producing a halogen-substituted benzene dimethanol, Japanese Unexamined Patent Application Publication No. 2007-224017 describes a method including reducing a halogen-substituted terephthalic acid with a borohydride compound. Japanese Unexamined Patent Application Publication Nos. 2007-211001, 2007-23006 and 2008-31158 describe a method for producing a halogen-substituted benzene dimethanol, the method including reducing a halogen-substituted terephthalic acid ester with a borohydride compound.

### DISCLOSURE OF THE INVENTION

The present application provides a novel method for producing a halogen-substituted benzene dimethanol.

The present application relates to the following inventions.
[1] A method for producing a halogen-substituted benzene dimethanol, the method comprising:
   a first step of reacting a compound represented by formula (1) 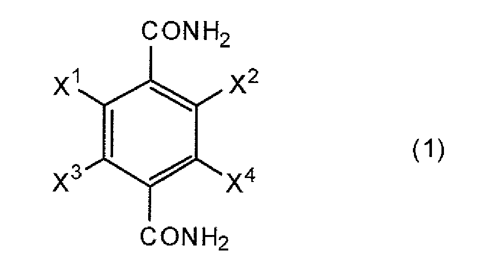 wherein X¹, X², X³ and X⁴ independently represent a hydrogen atom or a halogen atom, with the proviso that at least one of X¹, X², X³ and X⁴ is a halogen atom,
      with a compound represented by formula (2) 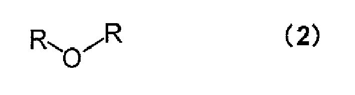 wherein R represents an optionally substituted alkoxycarbonyl group,
      to obtain a dicarboxamide compound represented by formula (3) 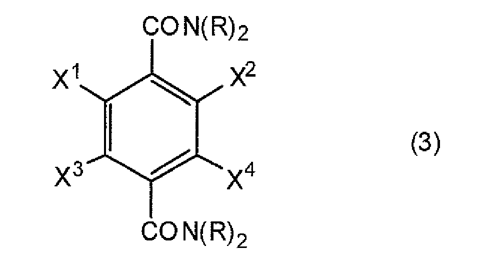 wherein R, X¹, X², X³ and X⁴ each represent the same meanings as described above; and
   a second step of reducing the above dicarboxamide compound with a borohydride compound to obtain a halogen-substituted benzene dimethanol represented by formula (4) 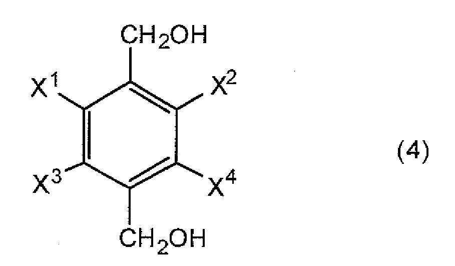
   wherein X¹, X², X³ and X⁴ each represent the same meanings as described above.
[2] The method as described in [1], wherein X¹, X², X³ and X⁴ are all halogen atoms.
[3] The method as described in [1] or [2], wherein R is an alkoxycarbonyl group having 2 to 5 carbon atoms.
[4] The method as described in any one of [1] to [3], wherein the compound represented by formula (1) is reacted with the compound represented by formula (2) in the presence of a 4-dialkylaminopyridine.
[5] The method as described in any one of [1] to [4], wherein the dicarboxamide compound is reduced in the presence of a monoalcohol.
[6] The method as described in any one of [1] to [5], wherein the monoalcohol is an aliphatic monoalcohol having 1 to 5 carbon atoms.
[7] The method as described in any one of [1] to [6], wherein the amount of the borohydride compound is 1 to 3 mol per mol of the dicarboxamide compound.
[8] The method as described in any one of [1] to [7], wherein the borohydride compound is an alkali metal borohydride.
[9] A method for producing a dicarboxamide compound, the method comprising reacting a compound represented by formula (1) 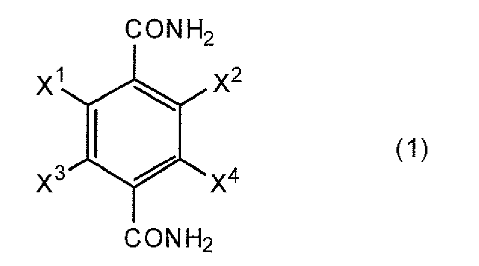 wherein X¹, X², X³ and X⁴ independently represent a hydrogen atom or a halogen atom, with the proviso that at least one of X¹, X², X³ and X⁴ is a halogen atom,
   with a compound represented by formula (2) 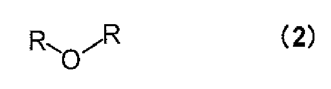 wherein R represents an optionally substituted alkoxycarbonyl group,
   to obtain a dicarboxamide compound represented by formula (3) 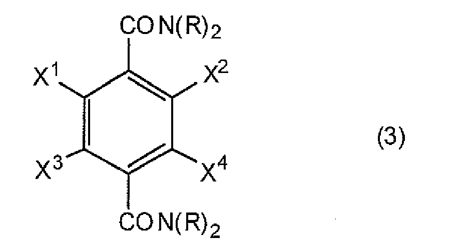 wherein R, X¹, X², X³ and X⁴ each represent the same meanings as described above.
[10] The method as described in [9], wherein X¹, X², X³ and X⁴ are all halogen atoms.
[11] The method as described in [9] or [10], wherein R is an alkoxycarbonyl group having 2 to 5 carbon atoms.
[12] The method as described in any one of [9] to [11], wherein the compound represented by formula (1) is reacted with the compound represented by formula (2) in the presence of a 4-dialkylaminopyridine.
[13] A method for producing a halogen-substituted benzene dimethanol represented by formula (4) 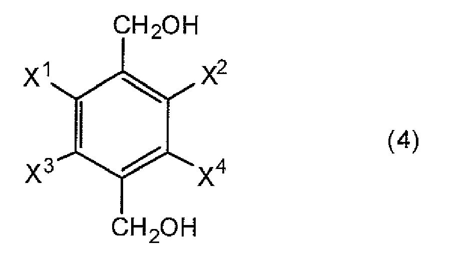 wherein X¹, X², X³ and X⁴ independently represent a hydrogen atom or a halogen atom, with the proviso that at least one of X¹, X², X³ and X⁴ is a halogen atom,
   the method comprising a step of reducing a dicarboxamide compound represented by formula (3) 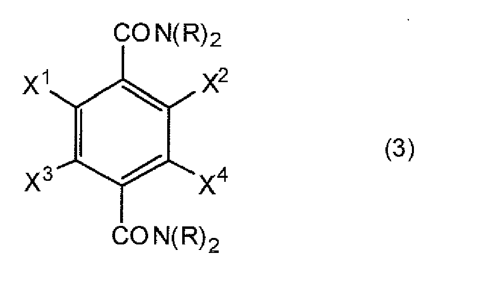 wherein X¹, X², X³ and X⁴ each represent the same meanings as described above, R represents an optionally substituted alkoxycarbonyl group,
   with a borohydride compound.
[14] The method as described in [13], wherein the dicarboxamide compound is reduced in the presence of a monoalcohol.
[15] The method as described in [13] or [14], wherein X¹, X², X³ and X⁴ are all halogen atoms.
[16] The method as described in any one of [13] to [15], wherein R is an alkoxycarbonyl group having 2 to 5 carbon atoms.
[17] The method as described in any one of [13] to [16], wherein the alkali metal borohydride is sodium borohydride.
[18] A dicarboxamide compound represented by formula (3) 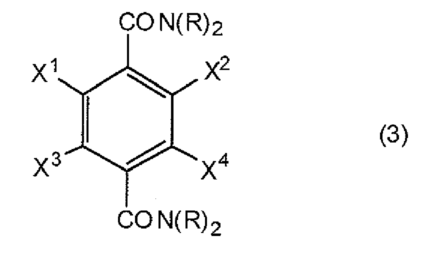 wherein R represents an optionally substituted alkoxycarbonyl group; and X¹, X², X³ and X⁴ independently represent a hydrogen atom or a halogen atom, with the proviso that at least one of X¹, X², X³ and X⁴ is a halogen atom.
[19] N,N,N',N'-tetrakis(tert-butoxycarbonyl)-(2,3,5,6-tetrafluoro benzene)-1,4-biscarboxamide.

### DETAILED DESCRIPTION OF THE INVENTION

The method for producing a halogen-substituted benzene dimethanol of the present invention includes a first step and a second step as described below.

The first step is a step of reacting a compound represented by formula (1) 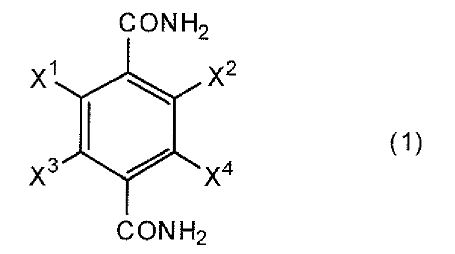 (hereinafter this compound may be referred to as Compound (1))
with a compound represented by formula (2) 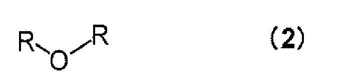 (hereinafter this compound may be referred to as Compound (2)) to obtain a dicarboxamide compound represented by formula (3)

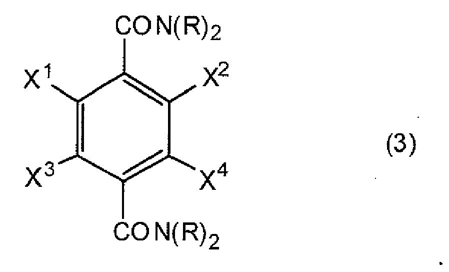

In formula (1), X¹, X², X³ and X⁴ independently represent a hydrogen atom or a halogen atom.

Examples of the above halogen atom include a fluorine atom, a chlorine atom and a bromine atom.

X¹, X², X³ and X⁴ are independently preferably a halogen atom, more preferably a fluorine atom and a chlorine atom, and further preferably a fluorine atom. It is preferred that X¹, X², X³ and X⁴ be all halogen atoms.

Examples of Compound (1) include 2-fluoroterephthalic acid diamide, 2-chloroterephthalic acid diamide,
2,5-difluoroterephthalic acid diamide,
2,6-difluoroterephthalic acid diamide,
2,3-difluoroterephthalic acid diamide,
2,6-dichloroterephthalic acid diamide,
2,3-dichloroterephthalic acid diamide,
2,3,5-trifluoroterephthalic acid diamide,
2,3,5-trichloroterephthalic acid diamide,
2,3,5,6-tetrafluoroterephthalic acid diamide,
2,3,5,6-tetrachloroterephthalic acid diamide and
2,3,5-trifluoro-6-chloroterephthalic acid diamide. Among these, 2,3,5,6-tetrafluoroterephthalic acid diamide is preferred.

Compound (1) can be produced according to, for example, a method in which a corresponding acid halide is reacted with ammonia gas (See, e.g., Zhurnal Obshchei Khimii, 34, 2953-2958 (1964)).

In formula (2), R represents an optionally substituted alkoxycarbonyl group, that is an alkoxycarbonyl group or a substituted alkoxycarbonyl group.

The alkoxycarbonyl group represented by R includes an alkoxycarbonyl group having 2 to 20 carbon atoms. Such an alkoxycarbonyl group may be linear, branched and cyclic, and examples thereof include a methoxycarbonyl group, an ethoxycarbonyl group, a propoxycarbonyl group, an isopropoxycarbonyl group, a n-butoxycarbonyl group, a tert-butoxycarbonyl group, an isobutoxycarbonyl group, a pentyloxycarbonyl group, a hexyloxycarbonyl group, a dodecyloxycarbonyl group, an octadecyloxycarbonyl group and a cyclohexyloxycarbonyl group.

Examples of substituents of the alkoxycarbonyl group include aryl groups such as a phenyl group and a naphthyl group. The substituted alkoxycarbonyl group includes an aryl-substituted alkoxycarbonyl group having 8 to 20 carbon atoms such as a benzyloxycarbonyl group.

In formula (2), R is preferably an alkoxycarbonyl group having 2 to 10 carbon atoms, more preferably an alkoxycarbonyl group having 2 to 5 carbon atoms, and further preferably a tert-butoxycarbonyl group.

Examples of Compound (2) include pyrocarbonic acid dimethyl ester, pyrocarbonic acid diethyl ester, pyrocarbonic acid diisopropyl ester, pyrocarbonic acid di-tert-butyl ester, and pyrocarbonic acid dibenzyl ester. Compound (2) may be used which is produced by a known method described in, for example, Japanese Unexamined Patent Application Publication No. Hei-4-211634 or which is commercially available.

In general, the amount of Compound (2) can be 4 mol or more per mol of Compound (1). The upper limit of the amount is not particularly restricted and is preferably 10 mol or less per mol of Compound (1) for economic purposes.

The reaction of Compound (1) and Compound (2) is generally carried out in the presence of a 4-dialkylaminopyridine.

In the 4-dialkylaminopyridine, a dialkylamino group contains preferably alkyl having 1 to 5 carbon atoms, and more preferably alkyl having 1 to 3 carbon atoms.

The 4-dialkylaminopyridine includes 4-dimethylaminopyridine, 4-diethylaminopyridine, 4-di(n-propyl)aminopyridine, 4-di(n-butyl)aminopyridine and the like, and 4-dimethylaminopyridine is preferred because of easy availability.

A 4-dialkylaminopyridine which is commercially available can be used.

A 4-dialkylaminopyridine is used in generally 0.001 mol or more, preferably in a range from 0.001 to 0.1 mol, and more preferably 0.005 to 0.1 mol per mol of Compound (1).

The reaction of Compound (1) and Compound (2) is generally carried out in the presence of a solvent. Examples of the solvent include ether solvents such as methyl tert-butyl ether, tetrahydrofuran and diglyme; nitrile solvents such as acetonitrile and propionitrile; and aromatic hydrocarbon solvents such as toluene and xylene. In the reaction, the solvent may be used individually, or two or more thereof may also be used.

The solvent is preferably a nitrile solvent, more preferably an alkylnitrile solvent, and further preferably an alkylnitrile solvent having 1 to 5 carbon atoms. When two or more solvents are used, it is preferred that a mixture of a nitrile solvent and an aromatic hydrocarbon solvent be used.

When a solvent is used, the amount of the solvent is not restricted and is generally 1 part by weight or more and 100 parts by weight or less per part by weight of Compound (1) for practical purposes.

The reaction temperature is generally in a range from -20° to 150°C, and preferably 30° to 100°C. Also, the reaction may be carried out under normal pressure conditions and the reaction may be carried out under pressurized conditions.

The progress of the reaction can be confirmed by general analytical means such as gas chromatography, high performance liquid chromatography, thin-layer chromatography, NMR, and IR.

The reaction of Compound (1) and Compound (2) is carried out by mixing these compounds and, if needed, heating the mixture. The order of mixing Compound (1) and Compound (2) is not particularly restricted. Even when the reaction is carried out in the presence of a solvent and a 4-dialkylaminopyridine, the order of mixing Compound (1), Compound (2), a solvent and a 4-dialkylaminopyridine is not particularly restricted.

A preferred embodiment of the reaction of Compound (1) and Compound (2) includes an aspect in which Compound (2) is added to a mixture of a solvent, a 4-dimethylaminopyridine and Compound (1) under reaction temperature.

A reaction mixture containing a dicarboxamide compound represented by formula (3) 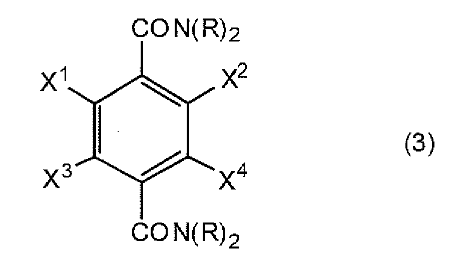 (hereinafter this compound may be referred to as Compound (3)) can be obtained by the reaction of Compound (1) and Compound (2).

In formula (3), R, X¹, X², X³ and X⁴ each have the same definitions as described above.

In formula (3), preferred ranges of X¹, X², X³ and X⁴ are the same as those of formula (1).

In formula (3), a preferred range of R is the same as that of formula (2).

Examples of Compound (3) include
N,N,N',N'-tetrakismethoxycarbonyl-(2-fluorobenzene)-1,4-bisc arboxamide,
N,N,N',N'-tetrakisethoxycarbonyl-(2-chlorobenzene)-1,4-bisca rboxamide,
N,N,N',N'-tetrakisbutoxycarbonyl-(2,5-difluorobenzene)-1,4-b iscarboxamide,
N,N,N',N'-tetrakisbenzyloxycarbonyl-(2,6-difluorobenzene)-1, 4-biscarboxamide,
N,N,N',N'-tetrakis(tert-butoxycarbonyl)-(2,3-difluorobenzene )-1,4-biscarboxamide,
N,N,N',N'-tetrakis(tert-butoxycarbonyl)-(2,5-dichlorobenzene )-1,4-biscarboxamide,
N,N,N',N'-tetrakis(tert-butoxycarbonyl)-(2,6-dichlorobenzene )-1,4-biscarboxamide,
N,N,N',N'-tetrakis(tert-butoxycarbonyl)-(2,3-dichlorobenzene )-1,4-biscarboxamide,
N,N,N',N'-tetrakis(tert-butoxycarbonyl)-(2,3,5-trifluorobenz ene)-1,4-biscarboxamide,
N,N,N',N'-tetrakis(tert-butoxycarbonyl)-(2,3,5-trichlorobenz ene)-1,4-biscarboxamide,
N,N,N',N'-tetrakismethoxycarbonyl-(2,3,5,6-tetrafluorobenzen e)-1,4-biscarboxamide,
N,N,N',N'-tetrakisethoxycarbonyl-(2,3,5,5-tetrafluorobenzene )-1,4-biscarboxamide,
N,N,N',N'-tetrakisisopropoxycarbonyl-(2,3,5,6-tetrafluoroben zene)-1,4-biscarboxamide,
N,N,N',N'-tetrakis(tert-butoxycarbonyl)-(2,3,5,6-tetrafluoro benzene)-1,4-biscarboxamide,
N,N,N',N'-tetrakisbenzyloxycarbonyl-(2,3,5,6-tetrafluorobenz ene)-1,4-biscarboxamide,
N,N,N',N'-tetrakismethoxycarbonyl-(2,3,5,6-tetrachlorobenzen e)-1,4-biscarboxamide,
N,N,N',N'-tetrakisethoxycarbonyl-(2,3,5,6-tetrachlorobenzene )-1,4-biscarboxamide,
N,N,N',N'-tetrakisisopropoxycarbonyl-(2,3,5,6-tetrachloroben zene)-1,4-biscarboxamide,
N,N,N',N'-tetrakis(tert-butoxycarbonyl)-(2,3,5,6-tetrachloro benzene)-1,4-biscarboxamide,
N,N,N',N'-tetrakisbenzyloxycarbonyl-(2,3,5,6-tetrachlorobenz ene)-1,4-biscarboxamide,
N,N,N',N'-tetrakismethoxycarbonyl-(2,3,5,6-tetrafluorobenzen e)-1,4-biscarboxamide,
N,N,N',N'-tetrakisethoxycarbonyl-(2,3,5,6-tetrafluorobenzene )-1,4-biscarboxamide,
N,N,N',N'-tetrakisisopropoxycarbonyl-(2,3,5,6-tetrafluoroben zene)-1,4-biscarboxamide,
N,N,N',N'-tetrakis(tert-butoxycarbonyl)-(2,3,5,6-tetrafluoro benzene)-1,4-biscarboxamide,
N,N,N',N'-tetrakis(tert-butoxycarbonyl)-(2,3,5-trifluoro-6-c hlorobenzene)-1,4-biscarboxamide and the like. Among these,
N,N,N',N'-tetrakisalkoxycarbonyl-(2,3,5,6-tetrahalobenzene)-1,4-biscarboxamide is preferred, N,N,N',N'-tetrakis(C1-C5 alkoxy)carbonyl-(2,3,5,6-tetrahalobenzene)-1,4-biscarboxamid e is more preferred, and
N,N,N',N'-tetrakis(tert-butoxycarbonyl)-(2,3,5,6-tetrafluoro benzene)-1,4-biscarboxamide is further preferred.

The reaction mixture, per se, obtained by the reaction of Compound (1) and Compound (2) can be used for a second step described below, and Compound (3) isolated from the reaction mixture by performing crystallization, condensation, liquid separation and the like can be used for the second step describe below. The isolated Compound (3) may be used for the second step after purified by a general method such as crystallization or column chromatography.

Preferably Compound (3) is isolated from the above reaction mixture by liquid separation using an organic solvent and an acidic aqueous solution. More preferably, an organic solvent and an acidic aqueous solution are added to the reaction mixture, and the obtained mixture is stirred and then left to stand, and Compound (3) can be recovered from the obtained organic solvent layer. When the reaction mixture contains an organic solvent, liquid separation can be performed by adding only an acidic aqueous solution to the reaction mixture. The organic solvent may be a solvent which is not miscible with water. The organic solvent includes aromatic hydrocarbon compounds such as toluene; aliphatic hydrocarbon compounds such as ethyl acetate; and nitrile compounds such as acetonitrile. The acidic aqueous solution is preferably an aqueous solution of an inorganic acid such as hydrochloric acid or sulfuric acid, and more preferably hydrochloric acid.

In the method for producing a halogen-substituted benzene dimethanol of the present invention, the second step is a step of reducing Compound (3) with a borohydride compound to obtain a halogen-substituted benzene dimethanol represented by formula (4) 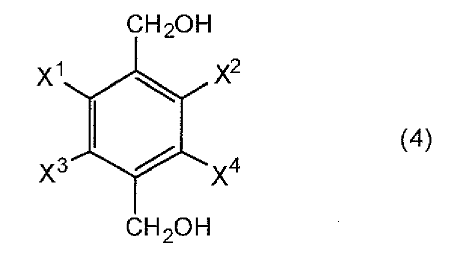 (wherein R, X¹, X², X³ and X⁴ each represent the same meanings as described above) (hereinafter this compound may be referred to as Compound (4)).

The borohydride compound includes an alkali metal borohydride, an alkaline earth metal borohydride and the like. Examples of the above alkali metal borohydride include sodium borohydride, lithium borohydride and potassium borohydride. The above alkaline earth metal borohydride includes calcium borohydride, magnesium borohydride and the like.

The borohydride compound is preferably an alkali metal borohydride, and more preferably sodium borohydride because of easy availability.

A borohydride compound may be used which is commercially available or which is prepared. Sodium borohydride, for example, can be easily prepared from a borate ester and sodium hydride. Also, other borohydride compounds can be prepared by reacting sodium borohydride with a corresponding metal halide. For example, calcium borohydride can be obtained by reacting sodium borohydride with calcium chloride. When a borohydride compound is prepared to be used, the borohydride compound which is prepared in advance may be used or it may be prepared simultaneously with the reduction reaction of Compound (3) in the presence of Compound (3).

The borohydride compound may be generally used in an amount of 1 mol or more per mol of Compound (3). The amount of the borohydride compound is generally 5 mol or less, and preferably in a range from 1 to 2.5 mol per mol of Compound (3) in respect of economy.

The reduction of Compound (3) is preferably carried out in the presence of a monoalcohol. When the reduction of Compound (3) is carried out in the presence of a monoalcohol, Compound (3) is efficiently converted to Compound (4). The above monoalcohol can be used as a solvent.

The above monoalcohol includes aliphatic monoalcohols having 1 to 4 carbon atoms such as methanol, ethanol, n-propanol, isopropanol, n-butanol and tert-butanol, and preferred is methanol.

The amount of the monoalcohol is not restricted and is generally in a range from 1 to 100 mol per mol of Compound (3).

The reduction of Compound (3) is generally carried out in the presence of a solvent. Examples of such solvent include ether solvents such as diethylether, methyl tert-butyl ether, tetrahydrofuran, dioxane and diisopropyl ether; and aromatic hydrocarbon solvents such as toluene, xylene and chlorobenzene. The above solvent is preferably an ether solvent.

The amount of the solvent is not restricted and is generally 1 part by mass or more and 100 parts by mass or less per part by mass of Compound (3). The reaction temperature is generally the range from 0° to 100°C, and preferably 20° to 70°C.

The reduction of Compound (3) is generally carried out by mixing Compound (3) and a borohydride compound in the presence of a solvent under reaction temperature conditions, and the order of mixing them is not particularly restricted. A preferred embodiment includes an aspect in which a monoalcohol is gradually added to a mixture containing Compound (3) and a borohydride compound. The reduction reaction of Compound (3) with a borohydride compound can be promoted by gradually adding a monoalcohol to the mixture.

This reaction is generally carried out under normal pressure conditions and may be carried out under pressurized conditions. The progress of the reaction can be confirmed by general analytical means such as gas chromatography and liquid chromatography.

After reaction termination, the obtained reaction mixture contains Compound (4) and to the mixture, an aqueous solution of a mineral acid, for example, hydrochloric acid, sulfuric acid, phosphoric acid, nitric acid or the like is added, and then Compound (4) can be isolated by known means such as neutralization, extraction and condensation, if needed. The obtained Compound (4) may be purified by a general methods such as crystallization or column chromatography.

The above Compound (4) is a compound represented by formula (4). In formula (4), preferred ranges of X¹, X², X³ and X⁴ are the same as those of formula (1).

The above Compound (4) includes 2-fluoro-1,4-benzene dimethanol, 2-chloro-1,4-benzene dimethanol,
2,5-difluoro-1,4-benzene dimethanol, 2,6-difluoro-1,4-benzene dimethanol, 2,3-difluoro-1,4-benzene dimethanol,
2,5-dichloro-1,4-benzene dimethanol, 2,6-dichloro-1,4-benzene dimethanol, 2,3-dichloro-1,4-benzene dimethanol,
2,3,5-trifluoro-1,4-benzene dimethanol,
2,3,5-trichloro-1,4-benzene dimethanol,
2,3,5,6-tetrafluorobenzene dimethanol,
2,3,5,6-tetrachlorobenzene dimethanol,
2,3,5-trifluoro-6-chlorobenzene dimethanol and the like. Among these, 2,3,5,6-tetrahalobenzene dimethanol is preferred,
2,3,5,6-tetrafluorobenzene dimethanol and
2,3,5,6-tetrachlorobenzene dimethanol are more preferred, and
2,3,5,6-tetrafluorobenzene dimethanol is further preferred.

A method for producing Compound (3), the method comprising reacting Compound (1) with Compound (2) to obtain Compound (3), is also one of the present inventions.

Compound (3) is useful as an intermediate in the method for producing a halogen-substituted benzene dimethanol. Compound (3) can be easily converted to a halogen-substituted benzene dimethanol. The method for producing Compound (3) is useful as a method to easily obtain Compound (3) suitable for producing a halogen-substituted benzene dimethanol.

A method for producing Compound (4), the method comprising reducing Compound (3) with a borohydride compound to obtain compound (4), is also one of the present inventions. Since the method for producing Compound (4) utilizes Compound (3), a halogen-substituted benzene dimethanol can be easily obtained.

### EXAMPLES

The present invention will be described in more detail below by way of examples, but the present invention is not limited thereto.

### Example 1

### (1) Preparation for

### N,N,N',N'-tetrakis(tert-butoxycarbonyl)-(2,3,5,6-tetrafluoro benzene)-1,4-biscarboxamide

Into a 100 mL flask equipped with a reflux condenser, 500 mg of 2,3,5,6-tetrafluoroterephthalic acid diamide, 10 g of toluene, 10 g of acetonitrile and 10 mg of 4-dimethylaminopyridine were charged at room temperature (about 25°C), and the internal temperature thereof was adjusted to 80°C. With stirring the contents therein at the same temperature, 3.0 g of pyrocarbonic acid di(tert-butyl ester) was added dropwise to the contents over 2 hours. After dropwise addition, the contents was stirred at the same temperature for 2 hours and then cooled to room temperature. To the obtained reaction mixture, 20 g of a 5% by weight aqueous hydrochloric acid solution was added, and the resulting mixture was stirred and then left to stand. The mixture was separated into two layers and liquid separation was performed to obtain an organic solvent layer, upper layer. The organic solvent layer was washed twice with 30 g of water, and from the resulting organic solvent layer, the solvent therein was distilled to obtain 1.3 g of {N,N,N',N'-tetrakis(tert-butoxycarbonyl)-(2,3,5,6-tetrafluor obenzene)-1,4-biscarboxamide as a pale yellow oily substance.
The yield was 96%.
N,N,N',N'-tetrakis(tert-butoxycarbonyl)-(2,3,5,6-tetra fluorobenzene)-1,4-biscarboxamide
¹H-NMR (δppm, CDCl₃, TMS standard) : 1.51 (s, 36H)

### (2) Preparation for 2,3,5,6-tetrafluorobenzene dimethanol

Into a 100 mL flask equipped with a reflux condenser, 125 mg of sodium borohydride and 5 g of tetrahydrofuran were charged at room temperature. To the mixture, 700 mg of the oily substance obtained from Example 1 (1) was added, and the internal temperature thereof was adjusted to 50°C, and then the contents therein were stirred at the same temperature for 2 hours. The obtained reaction mixture was cooled to room temperature, and to the mixture, 10 g of a 10% by weight aqueous hydrochloric acid solution and 10 g of ethyl acetate were added, and the resulting mixture was stirred and then left to stand. The mixture was separated into two layers and liquid separation was performed to obtain an organic solvent layer, upper layer. When the organic solvent layer was analyzed by a liquid chromatography internal standard method, the amount of 2,3,5,6-tetrafluorobenzene dimethanol obtained was 95 mg and the yield was 41%.

### Example 2

### (1) Preparation for

### N,N,N',N'-tetrakis(tert-butoxycarbonyl)-(2,3,5,6-tetrafluoro benzene)-1,4-biscarboxamide

Into a 100 mL flask equipped with a reflux condenser, 500 mg of 2,3,5,6-tetrafluoroterephthalic acid diamide, 10 g of acetonitrile and 10 mg of 4-dimethylaminopyridine were charged at room temperature, and the internal temperature thereof was adjusted to 80°C. With stirring the obtained mixture at the same temperature, 3. 0 g of pyrocarbonic acid di (tert-butyl ester) was added dropwise to the mixture over 2 hours. After dropwise addition, the obtained mixture was stirred at the same temperature for 2 hours, and then cooled to room temperature. To the obtained reaction mixture, 20 g of a 5% by weight aqueous hydrochloric acid solution and 20 g of ethyl acetate were added, and the resulting mixture was stirred and then left to stand. The mixture was separated into two layers and liquid separation was performed to obtain an organic solvent layer, upper layer. The organic solvent layer was washed twice with 30 g of water, and from the resulting organic solvent layer, the solvent therein was distilled to obtain a pale yellow oily substance, whose major component was

### N,N,N',N'-tetrakis(tert-butoxycarbonyl)-(2,3,5,6-tetrafluoro benzene)-1,4-biscarboxamide.

### (2) Preparation for 2,3,5,6-tetrafluorobenzene dimethanol

Into a 100 mL flask equipped with a reflux condenser, 240 mg of sodium borohydride and 10 g of tetrahydrofuran were charged at room temperature. To the mixture, the total amount of the oily substance obtained from Example 2 (1) was added and the internal temperature thereof was adjusted to 50°C, and then the obtained mixture was stirred at the same temperature for 2 hours. To the mixture, 5 g of methanol was added dropwise over 1 hour, and the obtained mixture was stirred at the same temperature for 1 hour. The obtained reaction mixture was cooled to room temperature, and to the mixture, 20 g of a 10% by weight aqueous hydrochloric acid solution and 20 g of ethyl acetate were added, and the resulting mixture was stirred and then left to stand. The mixture was separated into two layers and liquid separation was performed to obtain an organic solvent layer, upper layer. When the organic solvent layer was analyzed by a liquid chromatography internal standard method, the amount of 2,3,5, 6-tetrafluorobenzene dimethanol obtained was 360 mg. The overall yield from Example 2-1 was 81%.

### Example 3

Into a 100 mL flask equipped with a reflux condenser, 500 mg of 2,3,5,6-tetrafluoroterephthalic acid diamide, 5 g of acetonitrile and 10 mg of 4-dimethylaminopyridine were charged at room temperature, and the internal temperature thereof was adjusted to 60°C. With stirring the obtained mixture at the same temperature, 3.0 g of pyrocarbonic acid di (tert-butyl ester) was added dropwise to the mixture over 1 hour. After dropwise addition, the obtained mixture was stirred at the same temperature for 4 hours, and then cooled to room temperature. A crystal was deposited by the cooling. The crystal was filtered and washed with 1 ml of acetonitrile, and then dried to obtain 450 mg of a white crystal. This crystal was identified as N,N,N',N'-tetrakis(tert-butoxycarbonyl)-(2,3,5,6-tetrafluoro benzene)-1,4-biscarboxamide by NMR.
N,N,N',N'-tetrakis(tert-butoxycarbonyl)-(2,3,5,6-tetra fluorobenzene)-1,4-biscarboxamide
¹H-NMR (δppm, CDC13, TMS standard): 1.51 (s, 36H)
¹³C-NMR (δppm, CDCl₃, TMS standard): 27.5(s), 27.8(s), 86.0(s), 86.5(s), 148.2(s), 158.0(s)

### INDUSTRIAL APPLICABILITY

A halogen-substituted benzene dimethanol is an important compound as a raw material and an intermediate for medicine and pesticide. It is known that, in particular, 2,3,5,6-tetrafluorobenzene dimethanol is useful as an intermediate for a household pesticide (See, e.g., Japanese Patent Publication No. 2606892). The present invention is useful in such a method for producing a halogen-substituted benzene dimethanol and as the intermediate thereof.

## Claims

1. A method for producing a halogen-substituted benzene dimethanol, the method comprising:
a first step of reacting a compound represented by formula (1) 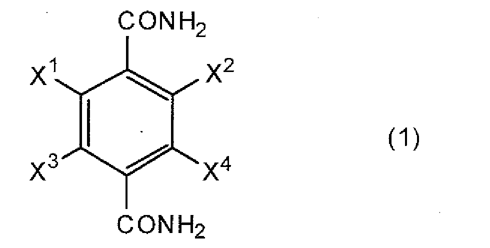 wherein X¹, X², X³ and X⁴ independently represent a hydrogen atom or a halogen atom, with the proviso that at least one of X¹, X², X³ and X⁴ is a halogen atom,
with a compound represented by formula (2) 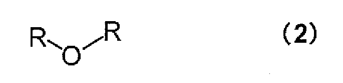 wherein R represents an optionally substituted alkoxycarbonyl group,
to obtain a dicarboxamide compound represented by formula (3) 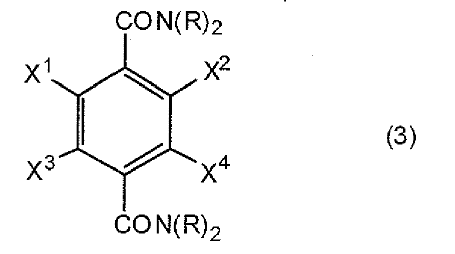 wherein R, X¹, X², X³ and X⁴ each represent the same meanings as described above; and
a second step of reducing said dicarboxamide compound with a borohydride compound to obtain a halogen-substituted benzene dimethanol represented by formula (4) 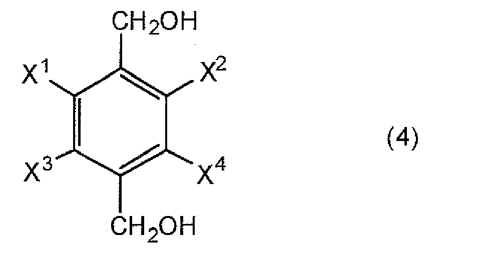 wherein X¹, X², X³ and X⁴ each represent the same meanings as described above.

2. The method according to claim 1, wherein X¹, X², X³ and X⁴ are all halogen atoms.

3. The method according to claim 1, wherein R is an alkoxycarbonyl group having 2 to 5 carbon atoms.

4. The method according to claim 1, wherein the compound represented by formula (1) is reacted with the compound represented by formula (2) in the presence of a 4-dialkylaminopyridine.

5. The method according to claim 1, wherein the dicarboxamide compound is reduced in the presence of a monoalcohol.

6. The method according to claim 5, wherein the monoalcohol is an aliphatic monoalcohol having 1 to 5 carbon atoms.

7. The method according to claim 1, wherein the amount of the borohydride compound is 1 to 3 mol per mol of the dicarboxamide compound.

8. The method according to claim 1, wherein the borohydride compound is an alkali metal borohydride.

9. A method for producing a dicarboxamide compound, the method comprising reacting a compound represented by formula (1) 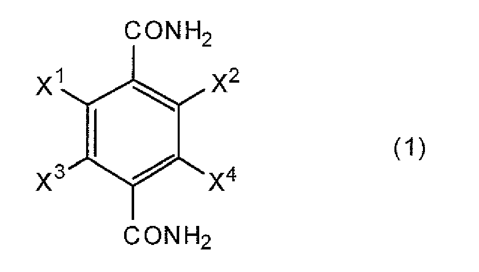 wherein X¹, X², X³ and X⁴ independently represent a hydrogen atom or a halogen atom, with the proviso that at least one of X¹, X², X³ and X⁴ is a halogen atom,
with a compound represented by formula (2) 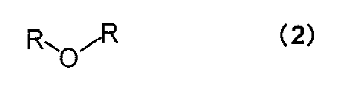 wherein R represents an optionally substituted alkoxycarbonyl group,
to obtain a dicarboxamide compound represented by formula (3) 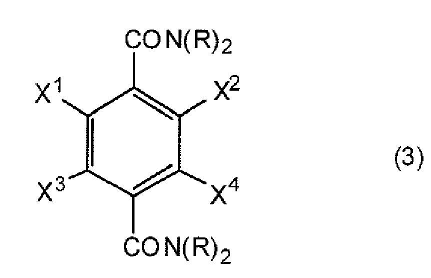 wherein R, X¹, X², X³ and X⁴ each represent the same meanings as described above.

10. The method according to claim 9, wherein X¹, X², X³ and X⁴ are all halogen atoms.

11. The method according to claim 9, wherein R is an alkoxycarbonyl group having 2 to 5 carbon atoms.

12. The method according to claim 9, wherein the compound represented by formula (1) is reacted with the compound represented by formula (2) in the presence of a 4-dialkylaminopyridine.

13. A method for producing a halogen-substituted benzene dimethanol represented by formula (4) 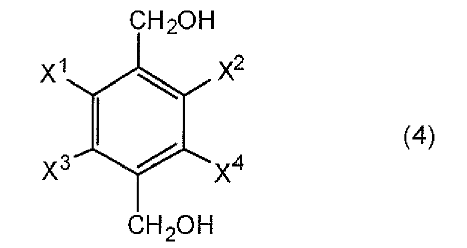 wherein X¹, X², X³ and X⁴ independently represent a hydrogen atoms or a halogen atom, with the proviso that at least one of X¹, X², X³ and X⁴ is a halogen atom,
the method comprising a step of reducing a compound represented by formula (3) 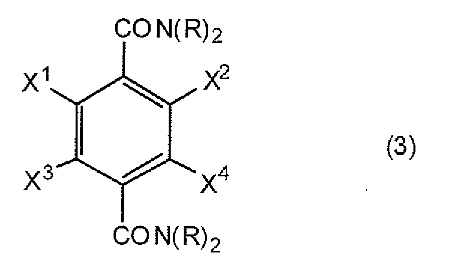 wherein X¹, X², X³ and X⁴ each represent the same meanings as described above, R represents an optionally substituted alkoxycarbonyl group,
with a borohydride compound.

14. The method according to claim 13, wherein the dicarboxamide compound is reduced in the presence of a monoalcohol.

15. The method according to claim 13, wherein X¹, X², X³ and X⁴ are all halogen atoms.

16. The method according to claim 13, wherein R is an alkoxycarbonyl group having 2 to 5 carbon atoms.

17. The method according to claim 13, wherein the alkali metal borohydride is sodium borohydride.

18. A dicarboxamide compound represented by formula (3) 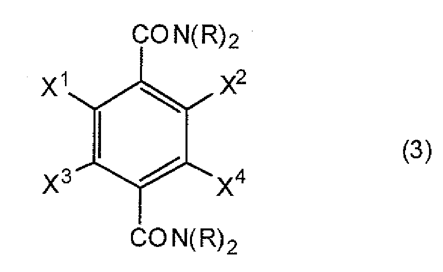 wherein R represents an optionally substituted alkoxycarbonyl group; and X¹, X², X³ and X⁴ independently represent a hydrogen atom or a halogen atom, with the proviso that at least one of X¹, X², X³ and X⁴ is a halogen atom.

19. N,N,N',N'-tetrakis(tert-butoxycarbonyl)-(2,3,5,6-tetrafluoro benzene)-1,4-biscarboxamide.
